# EUROPEAN PATENT SPECIFICATION

(11) **EP 1 990 403 B1**
(45) Date of publication and mention of the grant of the patent: **22.03.2017**
(21) Application number: 07254716.9
(22) Date of filing: 05.12.2007
(51) Int. Cl.: C12M 3/00, C12M 1/26, C12M 3/04, C12M 1/22

(54) **Container and method for production of biomembrane**
Behälter und Verfahren zur Herstellung einer Biomembran
Conteneur et procédé de production de membrane biologique

(30) Priority: 11.05.2007 CN 200710098296
(43) Date of publication of application: 12.11.2008
(73) Proprietor: Hsu, Connie Hui-Ju, Yung-Kang City, Tainan, Taiwan (CN)
(72) Inventor: Hsu, Connie Hui-Ju, Yung-Kang City, Tainan, Taiwan (CN)
(74) Representative: van Dam, Vincent

(56) References cited:
- EP-A- 0 267 093
- WO-A-88/02686
- WO-A-2007/080600
- CN-C- 100 367 925
- FR-A- 2 833 608
- GB-A- 2 413 562
- JP-A- 2007 197 471
- US-A- 5 605 836
- US-A- 6 071 727
- US-A1- 2006 286 434
- US-A1- 2007 026 516
- US-B1- 6 204 051
- US-B1- 6 312 952

## Description

### BACKGROUND OF THE INVENTION

### 1. Field of the Invention

The present invention relates to a container and a method for production of a biomembrane and, more particularly, to a container and a method of using microorganisms to produce polymer forming a biomembrane.

### 2. Description of Related Art

On the market, there are various masks for the purpose of satisfying the need to maintain human skin condition. In general, non-woven fabric is used as a base of masks for containing various cosmetics and medicines. This kind of non-woven fabric is not a cloth knit by thread, but it is a fiber material without being knit by textile processes. Therefore, the non-woven fabric has properties of air permeability, hygroscopicity, durability, drug tolerance, insulativity, and so forth. However, due to good hygroscopicity of the non-woven fabric, various active cosmetics and medicines contained therein are exposed in a two-way direction. Hence, the active cosmetics and medicines easily decompose or evaporate so that the masks can not exhibit all their functions.

Currently, a biomembrane made of polymer produced by microorganisms has been developed. This kind of biomembrane can be processed into, for example, dietary fibers for eating, masks for skin maintenance, and so on. The biomembrance has high water absorption so as to carry large amounts of nutrients. If the biomembrane is manufactured into a mask, the mask can supply nutrition for skin. Besides, the biomembrane has lots of advantageous properties of, for example, having free functional groups which can absorb dirt of skin.

In order to avoid covering organs such as the eyes, the nose, the mouth etc. on the face of the user otherwise resulting in being incapable of watching, smelling, eating, or talking, masks made of conventional non-woven fabric or made of biomembrane are all processed to form predetermined holes so that the user aforementioned requirements can be satisfied. However, formation of the predetermined holes needs die cutting that incurs complex steps of manufacturing and edge impairment of masks or predetermined holes. Accordingly, the yield of the masks is leveled down so that the costs of the production for the masks are raised. US5,605,836 and GB2413652 disclose a container comprising a number of protrusions, the container being suitable for the cultivation of cells forming a biomembrane. US2006/0286434 relates to cellulose composites and methods of making such composites. US6,071,727 relates to methods for producing microbial cellulose pellicle gels. CN100367925C discloses a cellulose gel facial film and its method of production.

### SUMMARY OF THE INVENTION

In view of the abovementioned shortcomings, one object of the present invention is to provide a container for production of a biomembrane which does not need the shape thereof to be changed. Besides, the containers can be stacked up vertically so as to get more horizontal space for production. The vertically stacked-up containers still have good airflow thereinto.

Another object of the present invention is to provide a method for production of a biomembrane. Through microbial strains naturally producing polymers such as polysaccharide, polyglutamate etc. in a predetermined container, a biomembrane can be formed to have a shape according to the predetermined container. Additionally, when the biomembrane is processed into a mask, no processes for changing the biomembrane are required. Therefore, the yield of the masks is raised. In another way, the manufactured mask can be used for skin maintenance and moisture supply to skin.

In order to achieve the object, a container for culturing a biomembrane is provided in accordance with present claim 1, which includes a bottom plate; a sidewall as defined in claim 1 disposed on the bottom plate and surrounding the periphery of the bottom plate to form an inner space; and at least one protrusion located on the surface of the bottom plate wherein a plurality of the containers are capable of being vertically stacked by insertion of the pins into the holes, wherein a vertical gap is formed between the stacked containers to ventilate the containers, wherein plural recesses are located on the outer surface of the sidewall; and wherein there are four protrusions arranged in a facial pattern corresponding to eyes, mouth and nose for a human facial mask.

In the abovementioned container, the sidewall may have a recessive brink and a prominent brink, the recessive brink is at the upper part of the sidewall, and the prominent brink is at the lower part of the sidewall. Besides, plural holes are defined in the upper surface of the sidewall, and plural pins corresponding to the holes are placed on the lower surface of the sidewall. If the containers are vertically stacked up one by one, horizontal space for culturing can be economized. Additionally, through the pins inserting into the holes or the prominent brink corresponding to the recessive brink, the containers stacked up can be more stable and not easily be loosened to collapse. The depth of the holes can be equal to or less than the height of the pins. Furthermore, the cross-section of the upper part of the pins can be equal to or greater than that of the pins.

Moreover, plural recesses locate on the outer surface of the sidewall, and those are not limited in any shape. The shape of the sidewall surrounding the periphery of the bottom plate is not limited to, but preferably is circular, elliptic, polygonal, irregular, or oval. The protrusion is not limited to, but preferably is columnar, taper, or sheeted. The protrusion can be arranged by way of corresponding to the facial features of a person, i.e. eyes, nose, mouth and so forth. The bottom plate can be planar or not planar, the latter means the bottom plate is a cambered surface.

In the abovementioned container, the protrusion can be formed by indenting from the outside surface of the bottom plate to the inside surface of the bottom plate. Hence, the material of the container can be economized by way of the above-mentioned method. Besides, the material of the container is not limited to, but preferably is transparent or nontransparent organic material (such as PVC, PP, and PE) or an anti-oxidative metal. The surface of the container can be selectively coated with PEP.

Furthermore, the present invention also discloses a method in accordance with claim 7 for production of a biomembrane comprising utilising a container for culturing a biomembrane according to claim 1, which includes the following steps:
(a) providing a first medium and a microbial strain therein, and shaking them to form a seed culture; (b) putting the seed culture into the container according to claim 1 having a second medium,; and (c) culturing the seed culture until a biomembrane having a predetermined thickness is formed, wherein the formed biomembrane has an integral human facial mask after being removed from the container.

In the abovementioned method of the present invention, the microbial strain preferably is *Acetobacter* spp., *Gluconacetobacter* spp., *Xanthomonas* spp., *or Bacillus* spp. More preferably, the microbial strain of the *Acetobacter* spp. is *A. xylinum;* the microbial strain of the 5 *Gluconacetobacter* spp. is *G xylinus* subsp. *Xylinus,* or *G Hansenii;* the microbial strain of the *Xanthomonas* spp. is *X camperastris\* and the microbial strain of the *Bacillus* spp. is *B. substilis var Natto.*

Additionally, the first medium and the second medium may comprise a carbohydrate, an N-containing compound, a mineral, and a growth cofactor. 10 The content of the carbohydrate, the N-containing compound, the mineral, and the growth cofactor preferably is 1-30%, 0.1-6%, 0.05-3%, and 0.05-2%, respectively. The carbohydrate is not limited to, but preferably is at least one selected from monosaccharide, disaccharides, polysaccharides, or carboxyl-containing compounds. The N-containing compound is not 15 limited to, but preferably is at least one selected from yeast extract, peptone, soybean powders, or gelatin. The mineral is not limited to, but preferably is at least one selected from chloride salt, ammonium salt, sulfide salt, potassium salt, phosphate, magnesium salt, or sodium salt. The growth cofactor is not limited to, but preferably is at least one selected from vitamin, 20 nicotinic acid, citric acid, or the derivatives thereof.

In order to obtain large-scaled seed culture for production, the method disclosed in the present invention can further comprise a step (al): amplifying the seed culture to afford great amount of seed culture for mass production after the step (a) is achieved.

The biomembrane manufactured by the method of the present invention can be processed into a facial mask, an eye mask, a chested mask, or a lip mask. The above-mentioned masks can efficiently provide water to maintain skin condition. Additionally, these masks are not required to change their shape by subsequent steps because the biomembrane in the present invention can be formed in the final shape. Furthermore, the biomembrane of the present invention does not have shortcomings of conventional masks being required to change shape thereof. Hence, the biomembrane in the present invention can have good yield without impairment due to the process of changing shape.

Other objects, advantages, and novel features of the invention will become more apparent from the following detailed description when taken in conjunction with the accompanying drawings.

### BRIEF DESCRIPTION OF THE DRAWINGS

FIG. 1a is a perspective view of a container for production of a biomembrane in the present invention;
FIG. 1b is a perspective view of a container when production of a biomembrane occurs in the example of the present invention;
FIG. 2 is a perspective view of a container when production of a biomembrane occurs in the example of the present invention;
FIG. 3 is a perspective view of a container when production of a biomembrane occurs in the example of the present invention;
FIG. 4 is a perspective view of a container when production of a biomembrane occurs in the example of the present invention;
FIG. 5 is a top view of a biomembrane produced from the examples of the present invention;
FIG. 6 is a chart of biocellulose production among examples 1 to 30 in the present invention; and
FIG. 7 is a chart of biocellulose production among examples 31 to 60 in the present invention.

### DETAILED DESCRIPTION OF THE PREFERRED EMBODIMENT

In the following examples, the microbial strain used in the present invention is *Acetobacter* spp., *Gluconacetobacter* spp., *Xanthomonas* spp., or *Bacillus* spp. In the *Acetobacter* spp., *A. xylinum* is used; in the *Acetobacter* spp., *G. xylinus* subsp. *Xylinus* ATTC 10821, *G. xylinus* subsp. *Xylinus* ATTC 700187, or *G*. *Hansenii* ATTC 23769 are used; in the *Xanthomonas* spp., *X camperastris* is used; and in the *Bacillus* spp., *B. substilis var Natto* is used. In the above mentioned, although *A. xylinum* is not named as *G xylinus,* they are the same microorganism.

The mediums used in the method of the present invention comprise a carbohydrate, an N-containing compound, a mineral, and a growth cofactor. In the following examples, the content of the carbohydrate is 1~30%; the content of the N-containing compound is 0.1~6%; the content of the mineral is 0.05~3%; and the content of the growth cofactor is 0.05~2%. Besides, at least one of monosaccharide, disaccharides, polysaccharides, and carboxyl-containing compounds is used as the carbohydrate; at least one of yeast extract, peptone, soybean powders, and gelatin is used as the N-containing compound; at least one of chloride salt, ammonium salt, sulfide salt, potassium salt, phosphate, magnesium salt, and sodium salt is used as the mineral; and at least one of vitamin, nicotinic acid, citric acid, and the derivatives thereof is used as the growth cofactor.

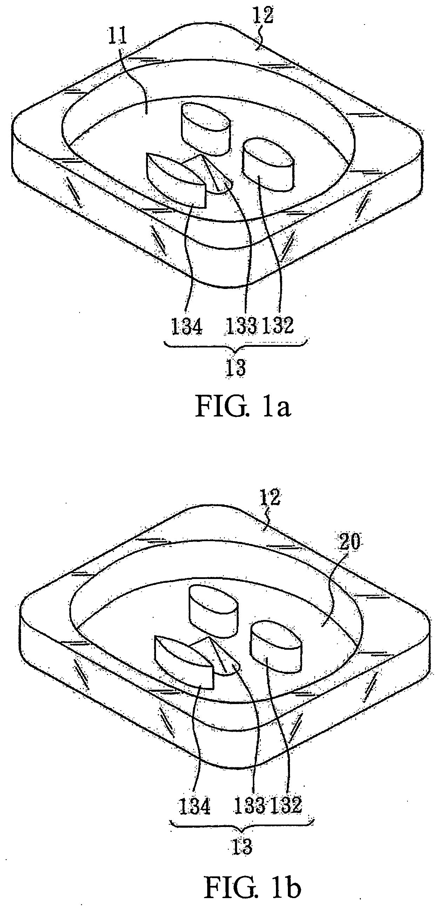

In the method of the present invention, which shape of the container is used is in view of the required biomembrane shape, for example, if a rectangular biomembrane is required, the cuboid container can be used, or if a circular biomembrane is required, the cylindrical container can be used. In the following examples, the container used therein is as shown in FIGs. 1a~4, but is not limited to. The container can be made of PVC, PP, PE, or antioxidative metals. Besides, the container can be selectively coated with PEP. PEP is a high molecular weight rust-preventing membrane, and that can prevent corrosive gas from permeating by coating the container and neutralizing the permeating corrosive gas.

### Examples 1-30

About 1~5 loops of *A. xylinum* are transferred from the slant culture to a sterile liquid medium (as the following Table 1). The liquid medium is cultured for 1~3 days at 37 °C by shaking, and then amplified with 2~30-folds sterile liquid medium. This amplification can be performed for 1~2 days in a fermenting tank or a shaking incubator so as to obtain seed culture for large-scaled production.

**Table 1**

| Components | Content (%) |
|---|---|
| Glucose | 2 |
| Peptone | 1 |
| Yeast extract | 1 |
| Citric acid | 0.1 |
| Sodium biphosphate | 0.3 |

The container for culturing used in the present examples is shown in FIG. 1a. The container a bottom plate 11; a sidewall 12 disposed on the bottom plate 11 and surrounding the periphery of the bottom plate 11, wherein an inner space is surrounded by the sidewall 12 and the bottom plate 11; and at least one protrusion 13 locating on the surface of the bottom plate 11. The protrusion 13 mentioned above can be in any shape, for example, the first protrusion 132 is an elliptic cylinder; the second protrusion 133 is a cone; and the third protrusion 133 is a leaf-shaped pillar. Besides, the protrusion 13 can be placed in any location in accordance with the shape of the required biomembrane.

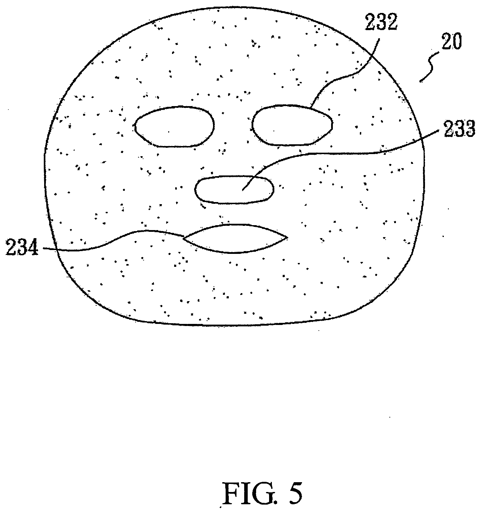

As shown in FIG. 1b, about 1%~50% of the seed culture is mixed with the culture medium in Table 2 and that is cultured in the inner space of the above-mentioned container. A biomembrane 20 is formed in a shape according to that of the container. At a constant temperature, the biomembrane 20 is cultured until it has a predetermined thickness. Then, the biomembrane 20 is taken out. As shown in FIG. 5, the biomembrane 20 have several openings 232, 233, and 234 respectively corresponding to the first protrusion 132, the second protrusion 133, and the third protrusion 133.

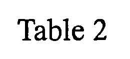

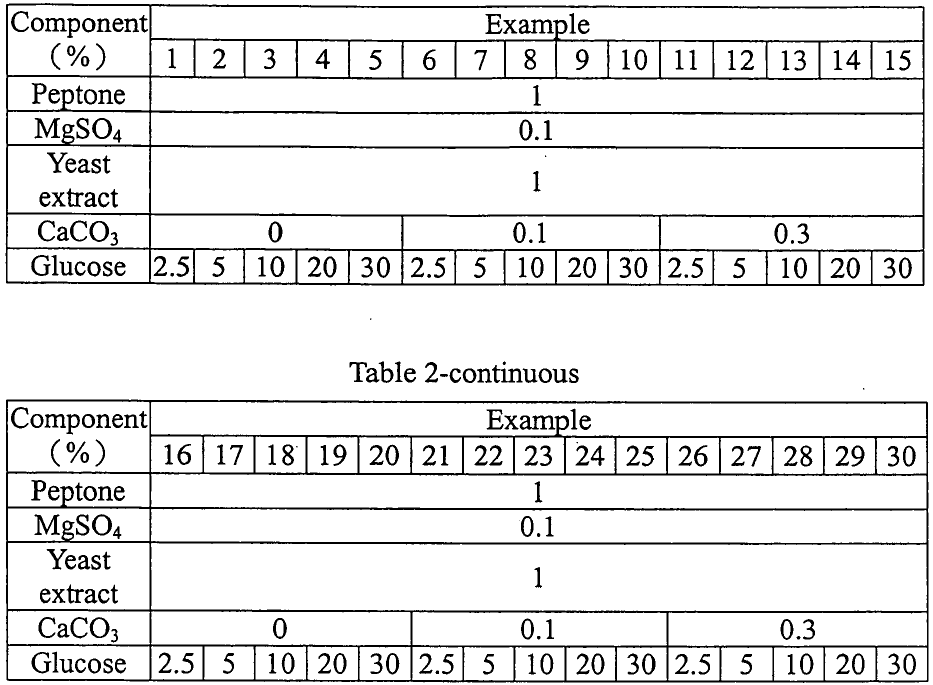

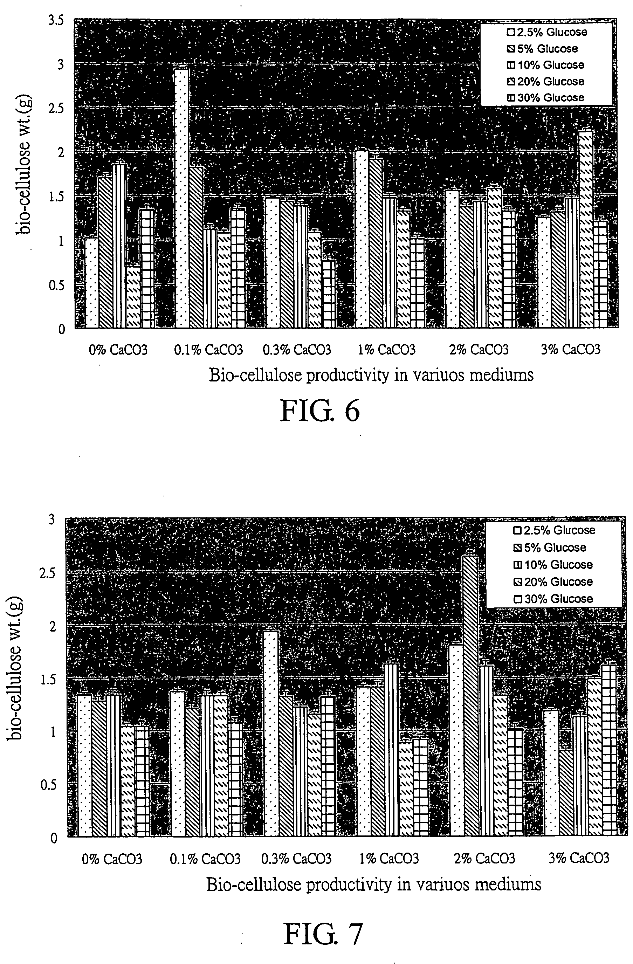

Through tests of the examples 1~30, the result is shown in FIG. 6. In accordance with the formation weight of the biomembrane 20, the culture medium comprising 1% yeast extract, 0.1 % CaCO₃, and 2.5% glucose can be an optimal medium to culture a biomembrane having an optimal production weight.

### Examples 31~60

About 1~5 loops of *A. xylinum* are transferred from the slant culture to a sterile liquid medium (as Table 1 in Examples 1~30). The liquid medium is cultured for 1~3 days at 37 °C by shaking, and then amplified with 2~30-folds sterile liquid medium. This amplification can be performed for 1~2 days in a fermenting tank or a shaking incubator so as to obtain seed culture for large-scaled production.

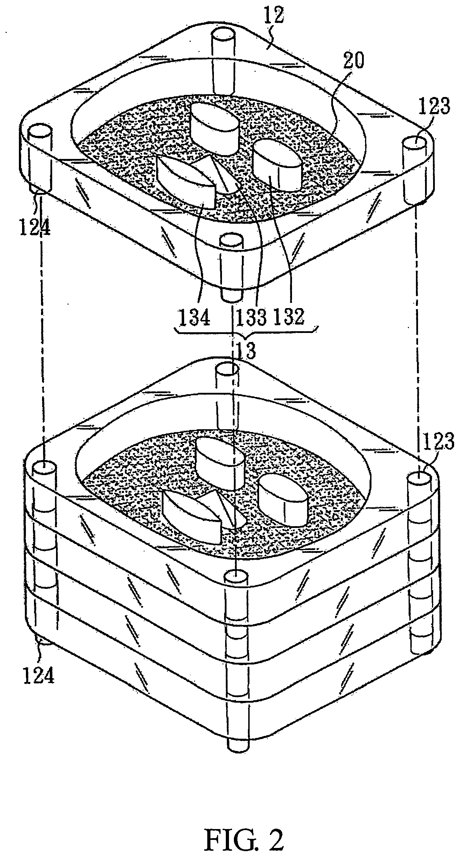

In the present examples, the container used therein is as shown in FIG. 2, and approximately is similar to the container shown in FIG. 1a. However, the container in the FIG. 2 has plural holes 123 through the upper surface of the sidewall 12, and plural pins 124 on the lower surface of the sidewall 12. The pins 124 are placed through corresponding holes 123.

About 1%~50% of the seed culture is mixed with the culture medium in Table 2 and that is cultured in the inner space of the above-mentioned container. Besides, the containers can be vertically stacked up one by one. Through the pins 124 inserting into the holes 123, the stacked up containers can become a stable vertical structure. Additionally, the stacked up containers does not easily collapse or become loosened. Hence, the horizontal space for production can be economized. A biomembrane 20 is formed in a shape according to that of the container. At a constant temperature, the biomembrane 20 is cultured until the biomembrane 20 have a predetermined thickness. Then, the biomembrane 20 as shown in FIG. 5 is taken out.

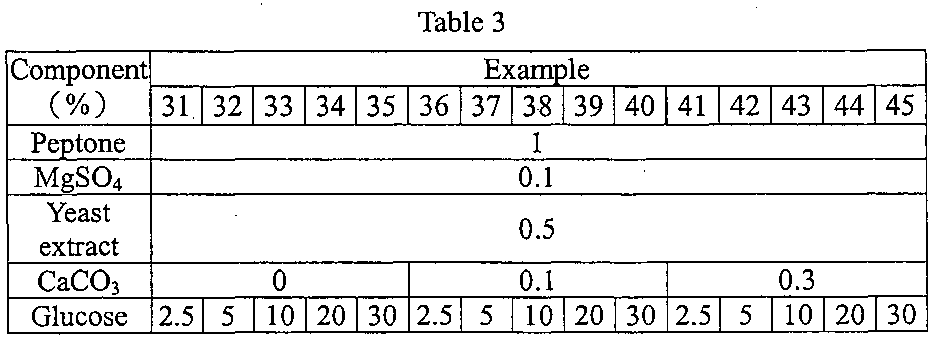

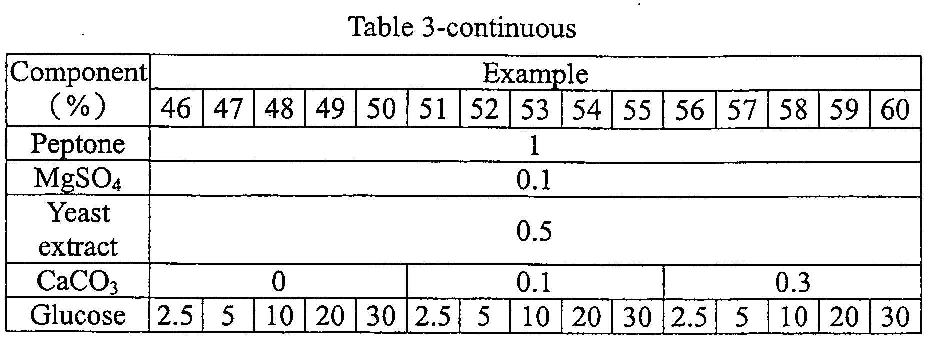

Through tests of the examples 31~60, the result is shown in FIG. 7. In accordance with the formation weight of the biomembrane 20, the culture medium comprising 0.5% yeast extract, 2 % CaCO₃, and 5% glucose can be an optimal medium to culture a biomembrane having a best production weight.

### Examples 61~63

About 1~5 loops of *G. xylinus* subsp. *Xylinus* ATTC 10821, *G. xylinus* subsp. *Xylinus* ATTC 700187, and *G. Hansenii* ATCC 23769 are transferred from the slant culture to a sterile liquid medium (as the following Table 4). The liquid medium is cultured for 1~3 days at 37 °C by shaking, and then amplified with 2~30-folds sterile liquid medium. This amplification can be performed for 1~2 days in a fermenting tank or a shaking incubator so as to obtain seed culture for large-scaled production.

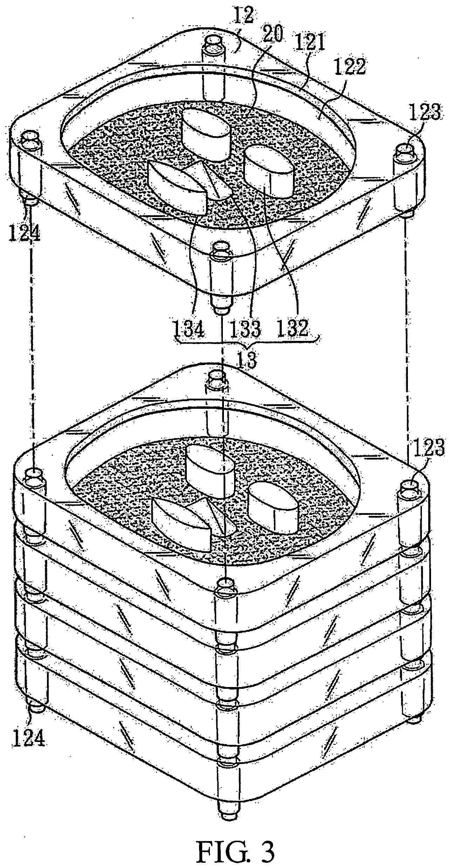

In the present examples, the container used therein is as shown in FIG. 3, and approximately is similar to the container shown in FIG. 2. However, the sidewall 12 of the container in the FIG. 3 has a recessive brink 121 at the upper part thereof, and a prominent brink 122 at the lower part thereof. Besides, the upper-portion cross-section of the pins 124 is greater than the cross-section of the holes 123.

About 1%~50% of the seed culture is mixed with the culture medium in Table 2 and that is cultured in the inner space of the above-mentioned container. Although FIG 3 shows that the recessive brink 121 and the prominent brink 122 are placed in the inside of the sidewall 12, those also can be placed in the outside of the sidewall 12. Hence, the container can be vertically stacked up one by one through the recessive brink 121 and the prominent brink 122 correspondingly inserted with each other.

Besides, the container also can be vertically stacked up one by one as shown in FIG. 3. Through the pins 124 inserting into the holes 123, there is a vertical gap between neighboring containers. Hence, air can ventilate the container to promote the biomembrane growth. A biomembrane 20 is formed in a shape according to that of the container. At a constant temperature, the biomembrane 20 is cultured until the biomembrane 20 has a predetermined thickness. Then, the biomembrane 20 as shown in FIG. 5 is taken out.

**Table 4**

| Component | Content (%) |
|---|---|
| Glucose | 2 |
| Peptone | 1 |
| Yeast extract | 1 |
| CaCO₃ | 0.5 |
| MgSO₄ | 0.1 |

### Example 64

About 1~5 loops of *X. campeastris* are transferred from the slant culture to a sterile liquid medium (as the following Table 5). The liquid medium is cultured for 1~3 days at 37 °C by shaking, and then amplified with 2~30-folds sterile liquid medium. This amplification can be performed for 1~2 days in a fermenting tank or a shaking incubator so as to obtain seed culture for large-scaled production.

**Table 5**

| Component | Content (%) |
|---|---|
| Glucose | 4 |
| Fructose | 2 |
| Peptone | 2 |
| Yeast extract | 2 |
| Citric acid | 0.2 |
| Potassium biphosphate | 0.2 |

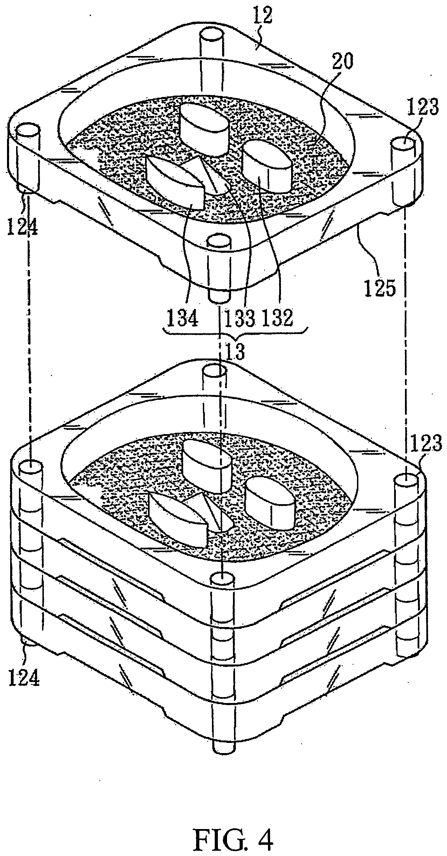

In the present examples, the container used therein is as shown in FIG. 4, and approximately is similar to the container shown in FIG. 2. However, the sidewall 12 has plural recesses 125 on the outer surface thereof. Besides, the recesses 125 are not limited to be formed on all the outer surface of the sidewall 12, but also can be formed on one, two, or three outer surfaces thereof.

About 1%~50% of the seed culture is mixed with the culture medium in Table 2 and that is cultured in the inner space of the above-mentioned container. Besides, the container also can be vertically stacked up one by one as shown in FIG. 3. Hence, air can ventilate the container to promote the biomembrane growth through the recesses 125. A biomembrane 20 is formed in a shape according to that of the container. At a constant temperature, the biomembrane 20 is cultured until the biomembrane 20 have a predetermined thickness. Then, the biomembrane 20 as shown in FIG. 5 is taken out.

### Example 65

About 1~5 loops of *B. subtilis var Natto* are transferred from the slant culture to a sterile liquid medium (as Table 5 of Example 65). The liquid medium is cultured for 1~3 days at 37 °C by shaking, and then amplified with 2~30-folds sterile liquid medium. This amplification can be performed for 1~2 days in a fermenting tank or a shaking incubator so as to obtain seed culture for large-scaled production.

In the present examples, the containers used therein are as shown in FIGs. 1~4. About 1%~50% of the seed culture is mixed with the culture medium in Table 2 and that is cultured in the inner space of the above-mentioned containers. A biomembrane 20 is formed in a shape according to that of the container. The biomembrane 20 is cultured at a constant temperature until the biomembrane 20 has a predetermined thickness. Then, the biomembrane 20 as shown in FIG. 5 is taken out.

The biomembranes prepared above in Example 1~65 are pretreated with alkaline solution, and treated with acidic solution. Then, the biomembranes are processed by conventional steps for biomembranes in the field such as boiling, washing etc. and then subjected to following processes of making masks such as sterilization, addition of efficient compositions, packaging, marking, and so on. Hence, biological masks with efficient compositions can be formed.

During manufacturing, the biomembranes made by the method of the present invention are not required to change in shape by subsequent steps such as molding, cutting, and so forth. Furthermore, the biomembrane of the present invention does not have shortcomings of conventional masks being required to change in shape. Hence, the biomembrane in the present invention can have good yield without impairment due to the process of changing shape. Additionally, the cost of manufacturing can be economized due to not generating any waste.

Because of the specific embodiments illustrating the practice of the present invention, a person having ordinary skill in the art can easily understand other advantages and efficiency of the present invention through the content disclosed therein.

## Claims

1. A container for culturing a biomembrane (20) comprising:
a bottom plate (11);
a sidewall (12) disposed on the bottom plate and surrounding and connecting the periphery of the bottom plate to form an inner space, wherein plural holes (123) are defined in the upper surface of the sidewall, and plural pins (124) corresponding to the holes are placed on the lower surface of the sidewall; and
at least one protrusion (13) located on the surface of the bottom plate,
wherein a plurality of the containers are capable of being vertically stacked by insertion of the pins into the holes, wherein a vertical gap is formed between the stacked containers to ventilate the containers, wherein plural recesses (125) are located on the outer surface of the sidewall; and wherein there are four protrusions (132, 133, 134) arranged in a facial pattern corresponding to eyes, mouth and nose for a human facial mask.

2. The container as claimed in claim 1, wherein the sidewall has a recessive brink (121) and a prominent brink (122), the recessive brink is at the upper part of the sidewall, and the prominent brink is at the lower part of the sidewall.

3. The container as claimed in claim 1, wherein the depth of the holes is less than or equal to the height of the pins.

4. The container as claimed in claim 1, wherein the area of the cross-section of the upper portion of the pins is greater than or equal to the area of the cross-section of the holes.

5. The container as claimed in claim 1, wherein the protrusion is columnar, taper, or sheeted and forms at least one void space in the biomembrane.

6. The container as claimed in claim 1, wherein the shape of the sidewall surrounding the periphery of the bottom plate is circular, elliptic, polygonal, irregular, or oval.

7. A method for the production of a biomembrane (20), comprising utilising a container for culturing a biomembrane according to claim 1 and further comprising the following steps:
(a) providing a first medium and a microbial strain therein, and shaking them to form a seed culture;
(b) putting the seed culture into the container according to claim 1 having a second medium; and
(c) culturing the seed culture until a biomembrane having a predetermined thickness is formed, wherein the formed biomembrane has an integral human facial mask after being removed from the container.

8. The method as claimed in claim 7, wherein the microbial strain is *Acetobacter* spp., *Gluconacetobacter* spp., *Xanthomonas* spp., or *Bacillus* spp.

9. The method as claimed in claim 8, wherein the microbial strain of the *Acetobacter* spp. is A. *xylinum.*

10. The method as claimed in claim 8, wherein the microbial strain of the *Gluconacetobacter* spp. is G. *xylinus* subsp. *Xylinus,* or G. *Hansenii.*

11. The method as claimed in claim 8, wherein the microbial strain of the *Xanthomonas* spp. is X. *camperastris.*

12. The method as claimed in claim 8, wherein the microbial strain of the *Bacillus* spp. is B. *substilis var Natto.*

13. The method as claimed in claim 7, wherein the first medium and the second medium comprise a carbohydrate, an N-containing compound, a mineral, and a growth cofactor.

14. The method as claimed in claim 13, wherein the content of the carbohydrate, the N-containing compound, the mineral, and the growth cofactor is 1~30%, 0.1~6%, 0.05~3%, and 0.05~2% respectively.

15. The method as claimed in claim 13, wherein the at least one carbohydrate is selected from the group consisting of monosaccharide, disaccharides, polysaccharides, and carboxyl-containing compounds.

16. The method as claimed in claim 13, wherein at least one N-containing compound is selected from the group consisting of yeast extract, peptone, soybean powders, and gelatin.

17. The method as claimed in claim 13, wherein at least one mineral is selected from the group consisting of chloride salt, ammonium salt, sulfide salt, potassium salt, phosphate, magnesium salt, and sodium salt.

18. The method as claimed in claim 13, wherein at least one growth cofactor is selected from the group consisting of vitamin, nicotinic acid, citric acid, and the derivatives thereof.

19. The method as claimed in claim 13, further comprising a step (a1):
amplifying the seed culture to afford great amount of seed culture for mass production after the step (a) is achieved.

## Patentansprüche

1. Ein Behälter zur Kultivierung einer Biomembran (20) umfassend:
eine Bodenplatte (11);
eine Seitenwand (12), die an der Bodenplatte angeordnet ist und den Umfang der Bodenplatte umgibt und verbindet, um einen inneren Raum zu bilden, wobei mehrere Löcher (123) in der oberen Oberfläche der Seitenwand festgelegt sind und mehrere Stifte (124), die den Löchern entsprechen, an der unteren Oberfläche der Seitenwand angeordnet sind;
und
mindestens ein Vorsprung (13), der an der Oberfläche der Bodenplatte angeordnet ist,
wobei eine Mehrzahl von Behältern dazu geeignet ist, vertikal durch Einführung der Stifte in die Löcher gestapelt zu werden, wobei ein vertikaler Abstand zwischen den gestapelten Behältern gebildet wird, um die Behälter zu belüften, wobei mehrere Vertiefungen (125) an der äußeren Oberfläche der Seitenwand angeordnet sind und wobei es vier Vorsprünge (132, 133, 134) gibt, die in Form eines Gesichtsmusters, entsprechend Augen, Mund und Nase für eine menschliche Gesichtsmaske, angeordnet sind.

2. Der Behälter wie in Anspruch 1 beansprucht, wobei die Seitenwand einen zurückgesetzten Rand (121) und einen hervortretenden Rand (122) aufweist, wobei sich der zurückgesetzte Rand am oberen Teil der Seitenwand befindet, und der hervortretende Rand sich am unteren Teil der Seitenwand befindet.

3. Der Behälter wie in Anspruch 1 beansprucht, wobei die Tiefe der Löcher geringer als oder gleich der Höhe der Stifte ist.

4. Der Behälter wie in Anspruch 1 beansprucht, wobei die Fläche des Querschnitts des oberen Teils der Stifte größer als oder gleich der Fläche des Querschnitts der Löcher ist.

5. Der Behälter wie in Anspruch 1 beansprucht, wobei der Vorsprung säulenartig, konisch oder plattenförmig ist und mindestens eine Aussparung in der Biomembran bildet.

6. Der Behälter wie in Anspruch 1 beansprucht, wobei die Form der Seitenwand, die den Umfang der Bodenplatte umgibt, rund, elliptisch, polygonal, irregulär oder oval ist.

7. Ein Verfahren für die Herstellung einer Biomembran (20) umfassend das Verwenden eines Behälters zur Kultivierung einer Biomembran gemäß Anspruch 1 und weiterhin umfassend die folgenden Schritte:
(a) Bereitstellen eines ersten Mediums und eines mikrobiellen Stamms darin und Schütteln derselben, um eine Saatkultur herzustellen;
(b) Platzieren der Saatkultur in dem Behälter gemäß Anspruch 1, der ein zweites Medium aufweist; und
(c) Kultivieren der Saatkultur, bis eine Biomembran mit einer vorgegebenen Dicke gebildet ist, wobei die gebildete Biomembran eine integrierte menschliche Gesichtsmaske aufweist, nachdem sie aus dem Behälter entfernt wurde.

8. Das Verfahren wie in Anspruch 7 beansprucht, wobei der mikrobielle Stamm *Acetobacterspp., Gluconacetobacterspp., Xanthomonas* spp. oder *Bacillus* spp. ist.

9. Das Verfahren wie in Anspruch 8 beansprucht, wobei der mikrobielle Stamm von *Acetobacter* spp. A. *xylinum* ist.

10. Das Verfahren wie in Anspruch 8 beansprucht, wobei der mikrobielle Stamm von *Gluconacetobacter* spp. G. *xylinus* subsp. *Xylinus* oder G.
*Hansenii* ist.

11. Das Verfahren wie in Anspruch 8 beansprucht, wobei der mikrobielle Stamm von *Xanthomonas* spp. X. *camperastris* ist.

12. Das Verfahren wie in Anspruch 8 beansprucht, wobei der mikrobielle Stamm von *Bacillus* spp. B. *substilis var Natto* ist.

13. Das Verfahren wie in Anspruch 7 beansprucht, wobei das erste Medium und das zweite Medium ein Kohlenhydrat, eine N-haltige Verbindung, ein Mineral und einen Wachstums-Cofaktor umfassen.

14. Das Verfahren wie in Anspruch 13 beansprucht, wobei der Gehalt des Kohlenhydrats, der N-haltigen Verbindung, des Minerals und des Wachstums-Cofaktors 1 ~ 30%, 0,1 ~ 6%, 0,05 ~ 3% beziehungsweise 0,05 ~ 2% beträgt.

15. Das Verfahren wie in Anspruch 13 beansprucht, wobei das mindestens eine Kohlenhydrat aus der Gruppe bestehend aus Monosacchariden, Disacchariden, Polysacchariden und carboxylhaltigen Verbindungen ausgewählt ist.

16. Das Verfahren wie in Anspruch 13 beansprucht, wobei mindestens eine N-haltige Verbindung aus der Gruppe bestehend aus Hefeextrakt, Pepton, Sojabohnenpulver und Gelatine ausgewählt ist.

17. Das Verfahren wie in Anspruch 13 beansprucht, wobei mindestens ein Mineral aus der Gruppe bestehend aus Chloridsalz, Ammoniumsalz, Sulfidsalz, Kaliumsalz, Phosphat, Magnesiumsalz und Natriumsalz ausgewählt ist.

18. Das Verfahren wie in Anspruch 13 beansprucht, wobei mindestens ein Wachstums-Cofaktor aus der Gruppe bestehend aus Vitamin, Nikotinsäure, Zitronensäure und Derivaten derselben ausgewählt ist.

19. Das Verfahren wie in Anspruch 13 beansprucht, weiterhin umfassend einen Schritt (a1):
Vervielfältigen der Saatkultur, um eine große Menge an Saatkultur zur Massenproduktion zu erzeugen, nachdem Schritt (a) vollendet wurde.

## Revendications

1. Récipient pour la culture d'une biomembrane (20) comprenant :
une plaque de fond (11) ;
une paroi latérale (12) agencée sur la plaque de fond et entourant et reliant la périphérie de la plaque de fond pour former un espace intérieur, dans lequel plusieurs trous (123) sont ménagés dans la surface supérieure de la paroi latérale, et plusieurs ergots (124) correspondant aux trous se situent sur la surface inférieure de la paroi latérale ; et
au moins une saillie (13) située sur la surface de la plaque de fond,
dans lequel une pluralité de récipients peuvent être empilés verticalement par insertion des ergots dans les trous, dans lequel un espace vertical subsiste entre les récipients empilés pour ventiler les récipients, dans lequel plusieurs évidements (125) se trouvent sur la surface extérieure de la paroi latérale ; et dans lequel il y a quatre protubérances (132, 133, 134) agencées selon un motif facial correspondant à des yeux, une bouche et un nez pour masque facial humain.

2. Récipient selon la revendication 1, dans lequel la paroi latérale comporte un bord récessif (121) et un bord proéminent (122), le bord récessif est dans la partie supérieure de la paroi latérale, et le bord proéminent dans la partie inférieure de la paroi latérale.

3. Récipient selon la revendication 1, dans lequel la profondeur des trous est inférieure ou égale à la hauteur des ergots.

4. Récipient selon la revendication 1, dans lequel la surface en coupe transversale de la partie supérieure des ergots est supérieure ou égale à la surface en coupe transversale des trous.

5. Récipient selon la revendication 1, dans lequel la protubérance est colonnaire, conique, ou en feuilles et forme au moins un espace de vide dans la biomembrane.

6. Récipient selon la revendication 1, dans lequel la forme de la paroi latérale entourant la périphérie de la plaque de fond est circulaire, elliptique, polygonale, irrégulière, ou ovale.

7. Procédé de production d'une biomembrane (20), comprenant l'utilisation d'un récipient pour la culture d'une biomembrane selon la revendication 1 et comprenant en outre les étapes suivantes :
(a) utilisation d'un premier milieu et d'une souche microbienne contenue dans celui-ci, et agitation pour former une culture d'ensemencement ;
(b) introduction de la culture d'ensemencement dans le récipient selon la revendication 1 contenant un second milieu ; et
(c) culture de la culture d'ensemencement jusqu'à ce qu'une biomembrane ayant une épaisseur prédéfinie soit formée, dans laquelle la biomembrane formée représente un masque facial humain intégral après son retrait du récipient.

8. Procédé selon la revendication 7, dans lequel la souche microbienne est *Acetobacter* spp., *Gluconacetobacter* spp., *Xanthomonas* spp., ou *Bacillus* spp.

9. Procédé selon la revendication 8, dans lequel la souche microbienne d'*Acetobacter* spp. est A. *xylinum.*

10. Procédé selon la revendication 8, dans lequel la souche microbienne de *Gluconacetobacter* spp. est G. *xylinus* subsp. *Xylinus,* ou G. *Hansenii.*

11. Procédé selon la revendication 8, dans lequel la souche microbienne de *Xanthomonas* spp. est X. *camperastris.*

12. Procédé selon la revendication 8, dans lequel la souche microbienne de *Bacillus* spp. est B. *subtilis var Natto.*

13. Procédé selon la revendication 7, dans lequel le premier milieu et le second milieu comprennent un glucide, un composé contenant de l'azote, un minéral, et un cofacteur de croissance.

14. Procédé selon la revendication 13, dans lequel la teneur en glucide, en composé contenant de l'azote, en minéral, et en cofacteur de croissance est de 1~30 %, 0,1~6 %, 0,05~3 %, et 0,05~2 %, respectivement.

15. Procédé selon la revendication 13, dans lequel ledit au moins un glucide est choisi dans le groupe constitué par le monosaccharide, les disaccharides, les polysaccharides, et les composés carboxylés.

16. Procédé selon la revendication 13, dans lequel au moins un composé contenant de l'azote est choisi dans le groupe constitué par l'extrait de levure, la peptone, les poudres de soja, et la gélatine.

17. Procédé selon la revendication 13, dans lequel au moins un minéral est choisi dans le groupe constitué par le sel de chlorure, le sel d'ammonium, le sel de sulfure, le sel de potassium, le phosphate, le sel de magnésium, et le sel de sodium.

18. Procédé selon la revendication 13, dans lequel au moins un cofacteur de croissance est choisi dans le groupe constitué par les vitamines, l'acide nicotinique, l'acide citrique, et leurs dérivés.

19. Procédé selon la revendication 13, comprenant en outre l'étape (a1) suivante :
amplification de la culture d'ensemencement pour obtenir une quantité importante de culture d'ensemencement à des fins de production de masse après que l'étape (a) est mise en oeuvre.
